(19) Europäisches Patentamt
European Patent Office
Office européen des brevets

(11) **EP 3 553 511 A1**

(12) **EUROPEAN PATENT APPLICATION**
published in accordance with Art. 153(4) EPC

(43) Date of publication:
16.10.2019 Bulletin 2019/42

(51) Int Cl.:
*G01N 27/416* (2006.01)    *C12M 1/34* (2006.01)
*C12Q 1/02* (2006.01)    *G01N 27/28* (2006.01)

(21) Application number: 17879398.0

(22) Date of filing: 29.11.2017

(86) International application number:
PCT/JP2017/042716

(87) International publication number:
WO 2018/105454 (14.06.2018 Gazette 2018/24)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 07.12.2016 JP 2016237241

(71) Applicant: Panasonic Intellectual Property
Management Co., Ltd.
Osaka-shi, Osaka 540-6207 (JP)

(72) Inventors:
• HIRAMOTO, Kaoru
Osaka-shi, Osaka 540-6207 (JP)
• USHIO, Hiroshi
Osaka-shi, Osaka 540-6207 (JP)
• YASUMI, Masahiro
Osaka-shi, Osaka 540-6207 (JP)

(74) Representative: Vigand, Philippe et al
Novagraaf International SA
Chemin de l'Echo 3
1213 Onex - Genève (CH)

(54) **ELECTROCHEMICAL MEASURING METHOD AND ELECTROCHEMICAL MEASURING DEVICE**

(57) A state of a biological sample is measured by the following method. An electrochemical measuring device including a working electrode and filled with a measuring liquid contacting the working electrode is prepared. A biological sample is put into the measuring liquid. A measuring potential is applied to the working electrode to measure a current value flowing through the working electrode. An oxidation potential is applied to the working electrode to oxidize a surface of the working electrode. This method allows an ambient state of the biological sample to be measured stably.

FIG. 3A

EP 3 553 511 A1

# FIG. 3B

**Description**

TECHNICAL FIELD

The present disclosure relates to an electrochemical measuring method and an electrochemical measuring apparatus for measuring and analyzing an activity state of, e.g. a cell aggregate or tissue.

BACKGROUND ART

Cells and tissues act while transporting and consuming various substances. For example, an embryo is divided while consuming oxygen in its surroundings. Accordingly, measurement of an ambient environment of a sample, such as cells or tissues, allows the activity state of the sample to be analyzed.

A method for measuring the ambient environment of a sample is to electrochemically measure substance concentrations in a solution containing the sample with an electrochemical measuring device including working electrodes (see, e.g. PTL 1).

CITATION LIST

PATENT LITERATURE

PTL 1: International Publication WO 2010/055942

SUMMARY

A state of a biological sample is measured by the following method. An electrochemical measuring device including a working electrode and a measuring liquid contacting the working electrode is prepared. A biological sample is put into the measuring liquid. A measuring potential is applied to the working electrode to measure a current value flowing through the working electrode. An oxidation potential is applied to the working electrode to oxidize a surface of the working electrode.

This method allows an ambient state of the biological sample to be measured stably.

BRIEF DESCRIPTION OF DRAWINGS

FIG. 1 is a perspective view of an electrochemical measuring device according to an exemplary embodiment.
FIG. 2 is a schematic view of an electrochemical measuring apparatus according to the embodiment.
FIG. 3A is a cross-sectional view of the electrochemical measuring device, along line 3A-3A shown in FIG. 1.
FIG. 3B is an enlarged top view of the electrochemical measuring device shown in FIG. 3A.

FIG. 4 is a flowchart illustrating an electrochemical measurement method according to the embodiment.
FIG. 5 is a diagram of an electric potential application protocol in the electrochemical measurement method shown in FIG. 4.
FIG. 6 is a diagram of another potential application protocol in the electrochemical measurement method shown in FIG. 4.
FIG. 7 is a diagram of still another potential application protocol in the electrochemical measurement method shown in FIG. 4.
FIG. 8 is a flowchart illustrating another electrochemical measurement method according to the embodiment.
FIG. 9 is a diagram of a potential application protocol in the electrochemical measurement method shown in FIG. 8.
FIG. 10 is a top view of another electrochemical measurement device according to the embodiment.
FIG. 11 is a flowchart illustrating still another electrochemical measurement method according to the embodiment.
FIG. 12 is a diagram of a potential application protocol in the electrochemical measurement method shown in FIG. 11.
FIG. 13A is an enlarged diagram of the potential application protocol in the electrochemical measurement method shown in FIG. 11.
FIG. 13B illustrates a potential applied to one working electrode in the potential application protocol shown in FIG. 13A.
FIG. 14A is an enlarged diagram of another potential application protocol in the electrochemical measurement method shown in FIG. 11.
FIG. 14B illustrates a potential applied to one working electrode in the potential application protocol shown in FIG. 14A.

DETAIL DESCRIPTION OF PREFERRED EMBODIMENT

Hereinafter, descriptions will be made regarding an electrochemical measuring method according to embodiments of the present disclosure, with reference to the accompanying drawings. It should be noted that each of the embodiments described below shows preferable and specific examples of the present disclosure. Numerical values, shapes, materials, constituent elements, and arrangements and connection modes of the constituent elements shown in the following embodiments are mere examples, and therefore are not intended to impose any limitation on the present disclosure. Moreover, of the constituent elements in the following exemplary embodiments, constituent elements not recited in an independent claim which defines the most generic concept of the present disclosure are described as optional constituent elements.

Throughout the drawings, figures are schematic ones and their illustrations are not necessarily strictly accurate. Throughout the figures, substantially identical elements are designated by the same numerals and symbols, and their duplicate explanations are omitted or simplified.

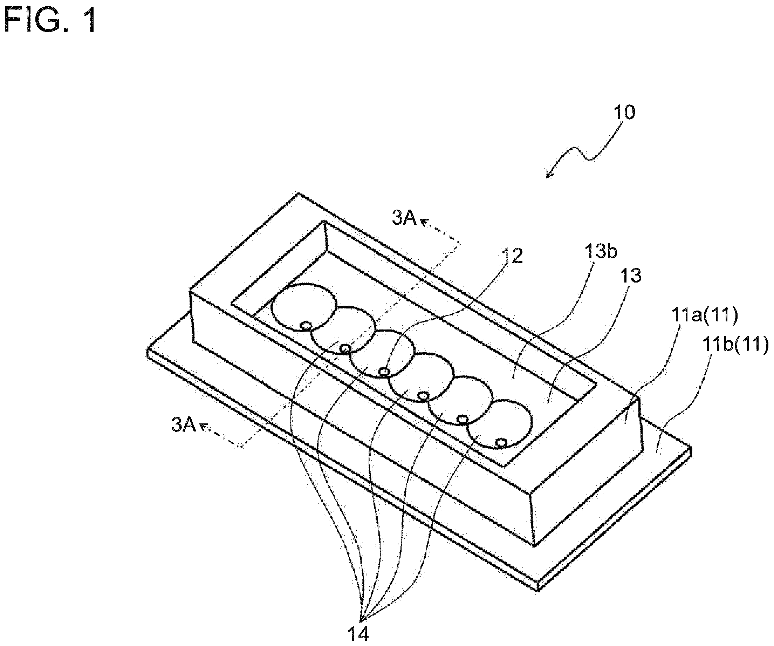

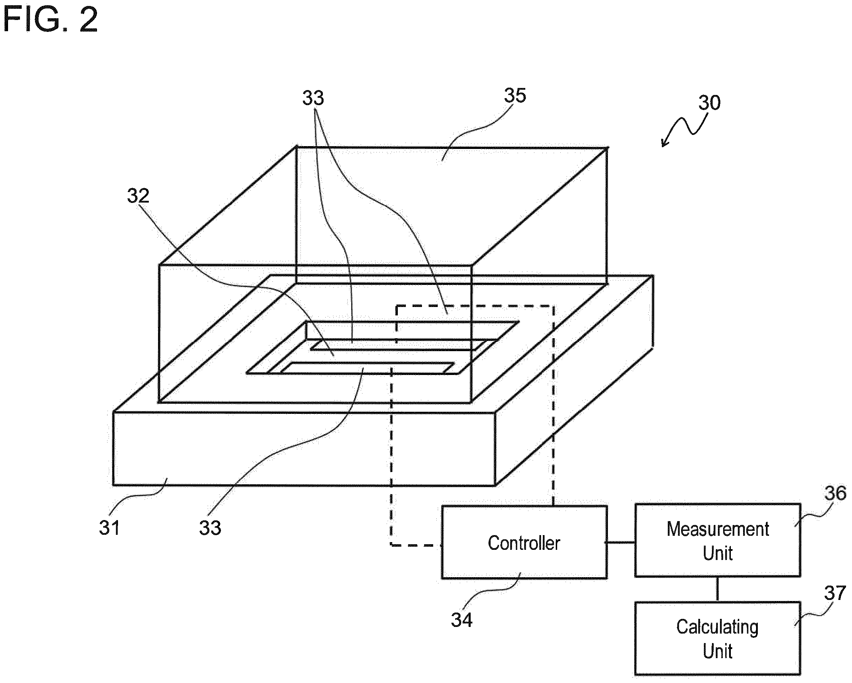

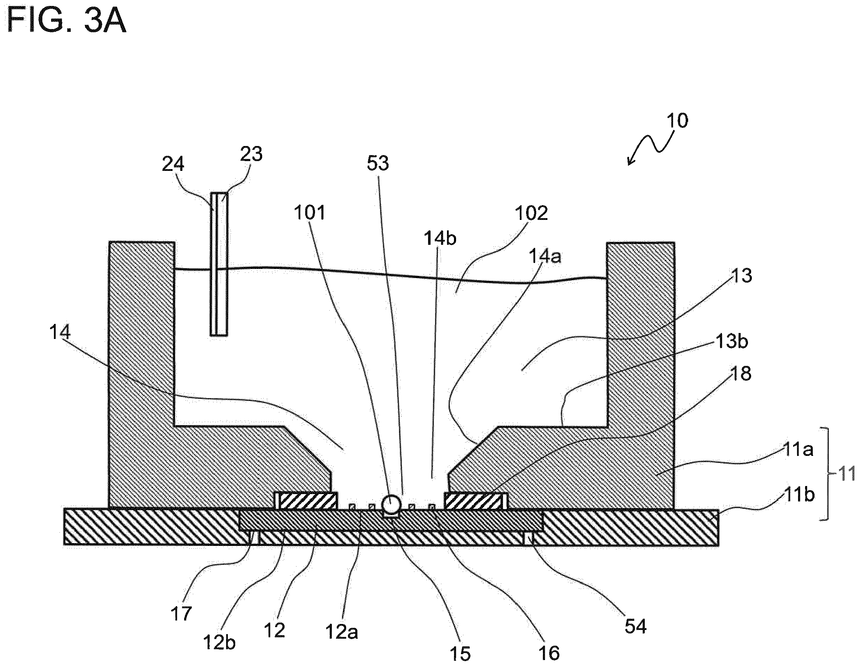

FIG. 1 is a perspective view of electrochemical measuring device 10 according to an exemplary embodiment. FIG. 2 is a schematic view of electrochemical measuring apparatus 30 according to the embodiment. FIG. 3A is a cross-sectional view of electrochemical measuring device 10 along 3A-3A shown in FIG 1 for schematically illustrating an operation of electrochemical measuring device 10.

Electrochemical measuring device 10 is a device for measuring an activity state of biological sample 101 that is a biological tissue or a cell aggregate, such as an embryo.

Electrochemical measuring device 10 includes container 11 and electrode chips 12. Container 11 includes upper container 11a and lower container 11b.

Container 11 includes electrode chips 12 mounted in container 11. Container 11 includes reservoir 13 for holding measuring liquid 102. Wells 14 having inverted-conical shapes are provided in bottom surface 13b of reservoir 13. Biological sample 101 is placed in each well 14. Container 11 is produced by, e.g. resin molding.

Each electrode chip 12 has upper surface 12a and lower surface 12b opposite to each other. Upper surface 12a of electrode chip 12 includes region 15. Working electrodes 16 are disposed on upper surface 12a of electrode chip 12. Region 15 is a mounting portion in which biological sample 101 is placed on upper surface 12a of electrode chip 12. Working electrodes 16 are used for electrochemical measurements of biological samples 101.

Region 15 serving as the mounting portion is implemented by, e.g. a recess provided in upper surface 12a of electrode chip 12. Region 15 may not be necessarily implemented by the recess provided in electrode chip 12. For example, region 15 may be implemented by a flat portion of upper surface 12a of electrode chip 12.

Working electrodes 16 surrounds region 15. Working electrodes 16 are disposed on upper surface 12a of electrode chip 12. Electrode chip 12 includes working electrodes 16 each of which surrounds region 15, and allows a constant distance between each working electrode 16 and biological sample 101 that is placed in region 15.

As shown in FIG. 2, electrochemical measuring apparatus 30 includes electrochemical measuring device 10, controller 34 connected to electrochemical measuring device 10, measurement unit 36 connected to controller 34, and calculating unit 37 connected to measurement unit 36.

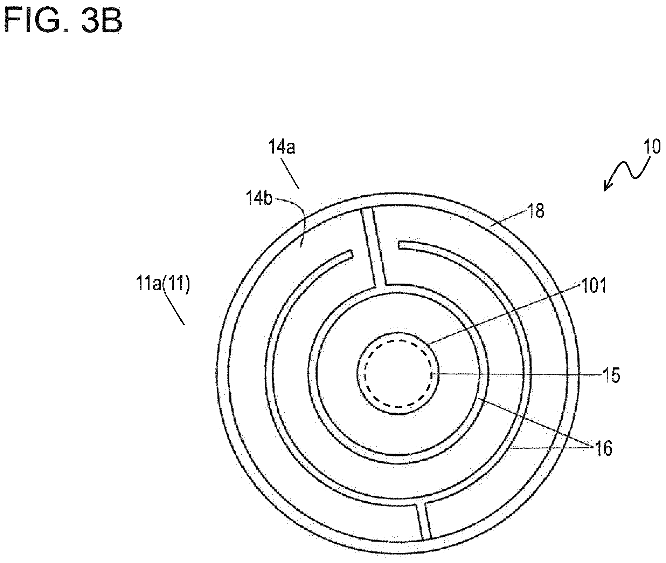

FIG. 3B is an enlarged top view of electrochemical measuring device 10 for showing bottom surface 14b of well 14. As shown in FIGs. 3A and 3B, plural working electrodes 16 are disposed such that the working electrodes substantially have concentric circular shapes about region 15, and are located at respective different distances from the center of region 15. This configuration allows electrochemical measuring device 10 to carry out an electrochemical measurement at plural different locations of different distances from biological sample 101.

In electrochemical measuring device 10, lower container 11b, electrode chip 12, and upper container 11a are stacked in this order. Well 14 has side wall surface 14a connected to bottom surface 13b of reservoir 13, and bottom surface 14b connected to side wall surface 14a. Through-hole 53 is provided in bottom surface 14b of well 14. Through-hole 53 passes through the lower part of upper container 11a. Working electrodes 16 of electrode chip 12 are exposed through through-hole 53 from bottom surface 14b of well 14. Electrode chip 12 is disposed below well 14.

Lower container 11b has through-holes 54 therein that are located below electrode chip 12. Connection terminals 17 electrically connected to respective working electrodes 16 on lower surface 12b of electrode chip 12. Connection terminals 17 are exposed via through-holes 54 to the lower surface of lower container 11b, i.e. the outside of electrochemical measuring device 10. Connection terminals 17 are connected to respective external measurement devices, such as controller 34 of electrochemical measuring apparatus 30.

Sealing member 18 is disposed between upper container 11a and electrode chip 12 to prevent measuring liquid 102 from leaking out.

Working electrodes 16 may be disposed within region 15. Moreover, connection terminals 17 may be disposed on a side surface of container 11.

Electrochemical measuring device 10 may have neither through-hole 53 formed in bottom surface 14b of well 14 nor electrode chip 12. In this case, region 15 and working electrodes 16 are disposed not on electrode chip 12 but directly on bottom surface 14b of well 14 of upper container 11a.

Electrochemical measuring apparatus 30 carries out the electrochemical measurement of biological sample 101 with electrochemical measuring device 10. Controller 34 of electrochemical measuring apparatus 30 applies a voltage to each of working electrodes 16 and measures an electric current that flows through working electrode 16.

Electrochemical measuring apparatus 30 further includes stage 31, mounting section 32, terminals 33, and cover 35.

Electrochemical measuring device 10 is disposed in mounting section 32 on stage 31. Mounting section 32 is a recess provided, e.g. in the upper surface of stage 31. Electrochemical measuring device 10 is fixed in the recess, i.e. mounting section 32.

Terminals 33 are provided on stage 31. Terminals 33 contacts respective connection terminals 17 of electrochemical measuring device 10. This configuration electrically connects working electrodes 16 to respective terminals 33. Moreover, terminals 33 are electrically connected to controller 34.

Controller 34 controls the magnitude of an electric potential applied to the working electrodes and the timing of applying the electric potential. Controller 34 includes a power supply, a potential applying circuit, etc. With this configuration, controller 34 generates a command signal to apply an electric potential, and applies the potential to the working electrodes.

Measurement unit 36 measures, e.g. an electric current that flows through working electrode 16 caused by the electric potential applied to the working electrode. Calculating unit 37 calculates, e.g. degree of activity of biological sample 101 based on the measured current value.

As shown in FIG. 2, controller 34, measurement unit 36, and calculating unit 37 may be implemented by circuits independent of one another. Alternatively, controller 34, measurement unit 36, and calculating unit 37 may be integrally configured with a one integrated circuit.

The electrochemical measuring apparatus may include cover 35 that covers mounting section 32. Cover 35 is disposed above stage 31. Cover 35 and stage 31 define a space in which electrochemical measuring device 10 is disposed. Cover 35 completely covers electrochemical measuring device 10 mounted in mounting section 32. Cover 35 keeps environment for the measurement of biological sample 101 in an appropriate state. That is, cover 35 produces the measurement environment isolated from the outside atmosphere. Cover 35 allows electrochemical measuring apparatus 30 to measure biological sample 101 in an appropriate environment. Such an appropriate environment is, for example, equivalent to the environment inside an incubator. The environment inside an incubator is, e.g. that the temperature is 37°C and the air contains 5% of carbon dioxide.

Electrochemical measuring apparatus 30 may be not necessarily include cover 35. In this case, the measurement can be carried out while stage 31 of electrochemical measuring apparatus 30 is placed in an incubator, with electrochemical measuring device 10 being mounted on the stage.

An operation of electrochemical measuring device 10 measuring biological sample 101 will be described with referring to FIG. 3A.

Biological sample 101 is, e.g. an embryo. Such an embryo includes a fertilized-egg having yet to be differentiated and a fertilized-egg having undergone cleavage.

An embryo undergoes cell division in a follicle while consuming oxygen that is present in its surroundings. By utilizing working electrodes 16, electrochemical measuring device 10 measures an amount of oxygen dissolved around the embryo in measuring liquid 102. Then, the measured amount of oxygen can be used to confirm the embryo's activity state based on its oxygen consumption.

Reference electrode 23 and counter electrode 24 are provided in reservoir 13.

Measuring liquid 102 is poured to fill reservoir 13 and well 14 such that the liquid contacts working electrodes 16, reference electrode 23, and counter electrode 24.

Working electrodes 16, reference electrode 23, and counter electrode 24 are electrically connected to controller 34 of electrochemical measuring apparatus 30.

Electrochemical measuring apparatus 30 may be a potentiostat. A potentiostat keeps electric potentials of working electrodes 16 constant with respect to reference electrode 23.

Biological sample 101 is placed in region 15 that is disposed on upper surface 12a of electrode chip 12.

When measuring an amount of oxygen dissolved in part of measuring liquid 102 locally around biological sample 101, the potentiostat applies an oxygen-reduction potential to each of working electrodes 16. Thus, the oxygen dissolved in measuring liquid 102 around working electrode 16 is reduced. The reduction of oxygen results in an electric current flowing through working electrode 16. The current flowing through working electrode 16 is measured by measurement unit 36.

The electric current value that flows through working electrode 16 has a correlation with the amount of oxygen dissolved in a part of measuring liquid 102 in the surroundings of working electrode 16. Therefore, the measurement of the current value that flows through working electrode 16 disposed at the surroundings of biological sample 101 detects a dissolved oxygen concentration around biological sample 101 in part of measuring liquid 102.

Counter electrode 24 may not necessarily be disposed. In this case, reference electrode 23 may function as counter electrode 24 besides the function of reference electrode 23.

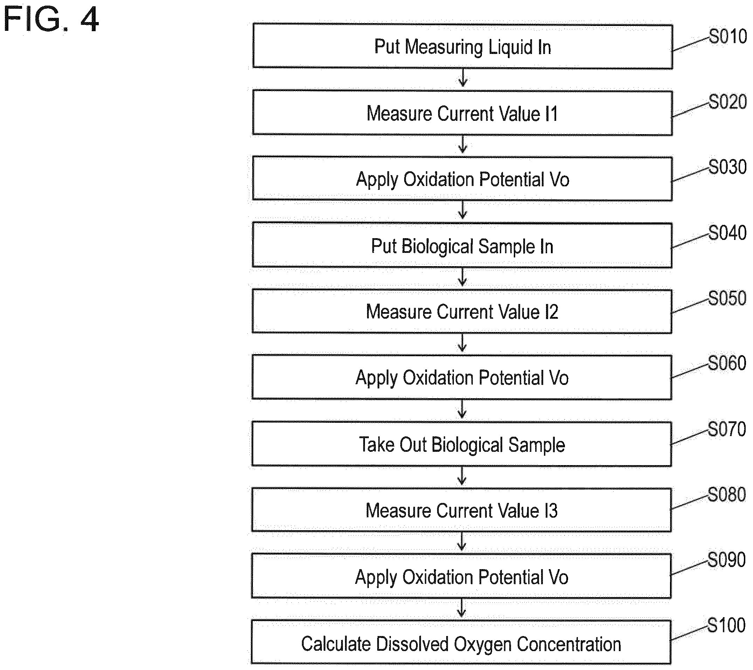

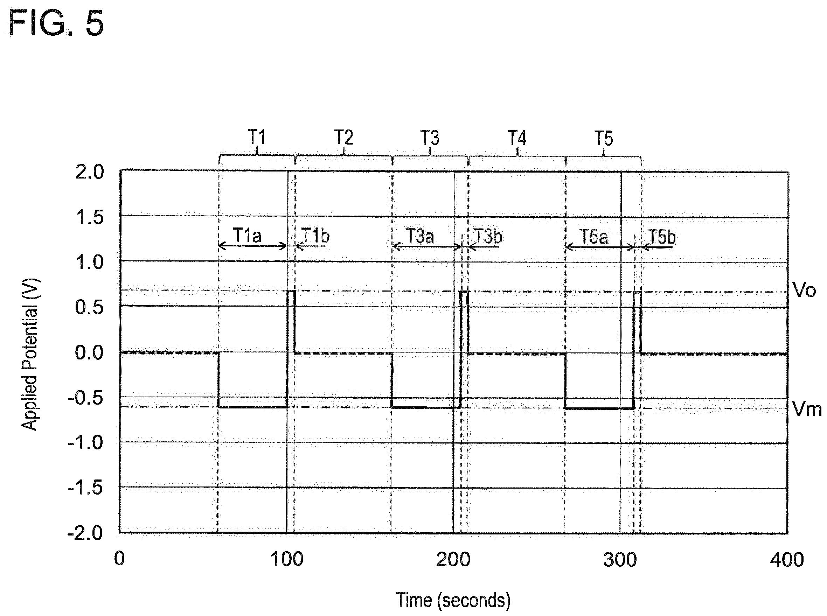

FIG. 4 is a flowchart illustrating an electrochemical measurement method according to the embodiment. FIG. 5 is a diagram of a potential application protocol that shows timings of applying potentials to each of working electrodes 16 in the electrochemical measurement method shown in FIG. 4. In FIG. 5, the vertical axis represents an electric potential applied to working electrode 16, and the horizontal axis represents time.

The electrochemical measuring method of measuring an amount of oxygen consumed by single biological sample 101, such as an embryo, will be described with referring to FIGs. 4 and 5. Here, single biological sample 101 is one cell, one cell aggregate, or one tissue. Plural cells contained and dispersed in measuring liquid 102 are excluded from single biological sample 101.

Plural working electrodes 16 are disposed on upper surface 12a of electrode chip 12 such that the working electrodes are located away from region 15 by different distances.

In the electrochemical measuring method shown in FIG. 4, measuring liquid 102 is put in container 11 (step S010). Then, measuring potential Vm is applied to each of working electrodes 16 to measure current value I1 in a blank state in which biological sample 101 is not put in it (step S020). Then, an oxidation potential is applied to working electrode 16 to oxidize a surface of working electrode 16 (step S030). Then, biological sample 101 is put in it (step S040). Then, measuring potential Vm is applied to working electrode 16 to measure current value I2 in a state after sample 101 is put in it (step S050). Then, an oxidation potential is applied to working electrode 16 to oxidize the surface of working electrode 16 (step S060). Then, biological sample 101 is taken out from it. (step S070) Then, measuring potential Vm is applied to working electrode 16 to measure current value 13 in a blank state after biological sample 101 is taken out. (step S080) Then, an oxidation potential is applied to working electrode 16 to oxidize the surface of working electrode 16 (step S090). After that, based on measured current values I1, I2, and I3, an amount of dissolved oxygen, i.e. a dissolved oxygen concentration, which is a concentration of the substance contained in measuring liquid 102 is calculated (step S100). Based on the calculated dissolved oxygen concentration, a degree of activity of biological sample 101 is measured.

Each of the above steps will be detailed below.

In step S010, measuring liquid 102 is put in reservoir 13 and wells 14 of container 11. Measuring liquid 102 contacts working electrodes 16, reference electrode 23, and counter electrode 24. Then, electrochemical measuring device 10 is mounted to mounting section 32 of electrochemical measuring apparatus 30. In this case, connection terminals 17 contact terminals 33, respectively. In accordance with the embodiment, platinum electrodes are employed as working electrodes 16 and counter electrode 24. In the description, a pseudo-reference electrode made of platinum is employed as reference electrode 23. However, the material of these electrodes is not limited to this. For example, the reference electrode may be a silver electrode or a silver chloride electrode.

In cases where measuring liquid 102 is previously put in container 11, the operation may start from step S020.

In step S020, before putting biological sample 101 into measuring liquid 102, measuring potential Vm is applied to working electrodes 16 during time period T1a within time period T1 shown in FIG. 5, thereby measuring current value I1 that flows through working electrode 16 in the state before the putting of biological sample 101. Measuring potential Vm is an oxygen-reduction potential. According to the embodiment, measuring potential Vm is a negative potential with respect to reference electrode 23. For example, measuring potential Vm is -0.6V. Measuring potential Vm is applied to working electrode 16 during period T1a of 40 seconds from the time point of 60 seconds to the time point of 100 seconds. The length of period T1a ranges, for example, from 10 seconds to 120 seconds. Measuring potential Vm applied to working electrode 16 causes reduction of the dissolved oxygen contained in a part of measuring liquid 102 around working electrode 16, causing an oxygen-reduction current to flow through working electrode 16. Electrochemical measuring apparatus 30 measures current value I1 of the oxygen-reduction current that flows through working electrode 16. It is noted, however, that the polarity, positive or negative, of measuring potential Vm changes depending on the substance to be measured. Measuring potential Vm may be determined to be a positive potential with respect to reference electrode 23. For example, when a concentration of hydrogen peroxide in measuring liquid 102 is measured, measuring potential Vm is set to be a positive potential with respect to reference electrode 23.

In step S020, current value I1 is measured which is attributed to an amount of dissolved oxygen, i.e. a dissolved oxygen concentration, contained in measuring liquid 102 in the blank state in which the measuring liquid is not influenced by biological sample 101.

Before period T1a, no potential is applied to working electrode 16. That is, working electrode 16 is floated electrically from reference electrode 23.

In step S030, oxidation potential Vo is applied to working electrode 16. Oxidation potential Vo is a potential to oxidize the surface of working electrode 16. Oxidation potential Vo is, e.g. +0.7V. Oxidation potential Vo is preferably smaller than the maximum value of a potential at and below which measuring liquid 102 is not electrolyzed, and preferably, is larger than the minimum value of a potential at and above which the surface of working electrode 16 is sufficiently oxidized. Oxidation potential Vo is preferably not smaller than 0.1 V and not larger than 1.3 V.

Oxidation potential Vo is applied to working electrode 16 during period T1b subsequent to period T1a. Period T1b is a period of four (4) seconds from the time point of 100 seconds to the time point of 104 seconds. This causes the surface of working electrode 16 to be oxidized. The oxidation of the surface of working electrode 16 allows the state of the surface of working electrode 16 to be held in a constant state. This configuration stabilizes measurements subsequently performed.

Period T1 is the sum of period T1a during which measuring potential Vm is applied and period T1b during which oxidation potential Vo is applied.

In step S040, biological sample 101 is put in electrochemical measuring device 10 during period T2 subsequent to period T1. Period T2 has a length of 60 seconds from the time point of 104 seconds to the time point of 164 seconds. The length of period T2 is equal to the time for an operator to put biological sample 101. For example, the length of period T2 ranges from 30 seconds to 120 seconds.

During period T2, working electrode 16 is floated electrically from reference electrode 23. In FIG. 5, the dashed line in period T2 indicates not a specific electric potential but a state in which working electrode 16 is floated electrically, that is, no potential is applied to working electrode 16. That is, biological sample 101 is put in measuring liquid 102 in period T2 during which no potential is applied to working electrode 16. At this moment, no current flows through working electrode 16, consequently preventing current noises attributed to the putting of biological sample 101. Even in cases where biological sample 101 accidentally contacts working electrode 16, no current flows through working electrode 16, thereby preventing biological sample 101 from suffering damage attributed to such a possible accidental current.

In step S050, during period T3a subsequent to period T2, measuring potential Vm is applied to working electrode 16, and current value I2 of an electric current that flows through working electrode 16 is measured while biological sample 101 is in measuring liquid 102 after biological sample 101 is put in it. Measuring potential Vm is an oxygen-reduction potential. According to the embodiment, measuring potential Vm is, e.g. -0.6V. Measuring potential Vm is applied to working electrode 16 during period T3a of 40 seconds from the time point of 164 seconds to the time point of 204 seconds. The length of period T3a is appropriately determined depending on the size and kind of biological sample 101. According to the embodiment, the length of period T3a ranges, e.g. from 10 seconds to 120 seconds. Measuring potential Vm applied to working electrode 16 causes reduction of the dissolved oxygen contained in a part of measuring liquid 102 around working electrode 16, thereby causing an oxygen-reduction current to flow through working electrode 16. Electrochemical measuring apparatus 30 measures current value I2 of the oxygen-reduction current that flows through working electrode 16.

In step S050, current value I2 is measured which is attributed to an amount of dissolved oxygen (a dissolved oxygen concentration) that is influenced by respiratory activity of biological sample 101.

Due to the respiratory activity, biological sample 101 mounted there consumes oxygen dissolved in measuring liquid 102 around the biological sample. Consequently, the concentration of the dissolved oxygen in measuring liquid 102 decreases around biological sample 101. The amount of the dissolved oxygen approaches an amount of saturated dissolved oxygen in measuring liquid 102 as the distance from biological sample 101 to the part of measuring liquid 102 increases.

The greater the respiratory activity of biological sample 101 is, the more the amount of oxygen around biological sample 101 is consumed. That is, the amount of respiratory activity of biological sample 101, i.e., the fertilized egg is determined by the gradient of oxygen concentration around biological sample 101.

In step S060, oxidation potential Vo is applied to working electrode 16 during period T3b of four (4) seconds which is subsequent to period T3a and lasts from the time point of 204 seconds to the time point of 208 seconds. Oxidation potential Vo is a potential to oxidize the surface of working electrode 16. Oxidation potential Vo is, e.g. +0.7V. The applied potential oxidizes the surface of working electrode 16. The oxidation of the surface of working electrode 16 allows the state of the surface of working electrode 16 to be held in a constant state. This results in a stable measurement that is subsequently performed.

Period T3 is the sum of period T3a during which measuring potential Vm is applied and period T3b during which oxidation potential Vo is applied.

In step S070, during period T4 subsequent to period T3, biological sample 101 is taken out from electrochemical measuring device 10, that is, biological sample 101 is taken out from measuring liquid 102. Period T4 has a length of 60 seconds lasting from the time point of 208 seconds to the time point of 268 seconds. The length of period T4 is equal to the time required for an operator to take out biological sample 101. For example, the length ranges from 30 seconds to 120 seconds.

During period T4, working electrode 16 is floated electrically from reference electrode 23. In FIG. 5, the dashed line in period T4 indicates not a specific electric potential but a state in which no potential is applied to working electrode 16. That is, in period T4, biological sample 101 is taken out from measuring liquid 102 while a potential is not applied to working electrode 16. At this moment, no current flows through working electrode 16, thereby preventing noises attributed to the taking out of biological sample 101. Even in cases where biological sample 101 accidentally contacts working electrode 16, no current flows through working electrode 16, thereby preventing biological sample 101 from suffering damage attributed to such a possible accidental current.

In step S080, during period T5a subsequent to period T4, measuring potential Vm is applied to working electrode 16, thereby measuring current value 13 of an electric current that flows through working electrode 16 after biological sample 101 is taken out from it. Period T5a has a length of 40 seconds lasting from the time point of 268 seconds to the time point of 308 seconds. Measuring potential Vm is an oxygen-reduction potential. According to the embodiment, measuring potential Vm is, e.g. -0.6V. The length of period T5a ranges, e.g. from 10 seconds to 120 seconds. Measuring potential Vm applied to working electrode 16 causes reduction of the dissolved oxygen contained in a part of measuring liquid 102 locally around working electrode 16, thereby causing an oxygen-reduction current to flow through working electrode 16. Electrochemical measuring apparatus 30 measures current value 13 of the oxygen-reduction current that flows through working electrode 16.

In step S080 current value 13 can be measured which is attributed to an amount of oxygen (a dissolved oxygen concentration) dissolved in measuring liquid 102 in the blank state in which the measuring liquid is not influenced by biological sample 101.

In step S090, during period T5b subsequent to period T5a, oxidation potential Vo is applied to working electrode 16. Period T5a has a length of four (4) seconds lasting from the time point of 308 seconds to the time point of 312 seconds. Oxidation potential Vo is a potential required to oxidize the surface of working electrode 16. Oxidation potential Vo is, e.g. +0.7V. The applied potential oxidizes the surface of working electrode 16. The oxidation of the surface of working electrode 16 allows the state of the surface of working electrode 16 to be held in a constant state. This results in a stable measurement that is subsequently performed.

Period T5 is the sum of period T5a during which measuring potential Vm is applied and period T5b during which oxidation potential Vo is applied.

After step S080, in cases where a measurement of a current value by applying an electric potential to working electrode 16 is not performed, step S090 is not required to be performed because of no need for holding the condition of surface of working electrode 16.

The values of measuring potentials Vm in steps S020, S050, and S080 are preferably identical to one another. Moreover, the values of oxidation potentials Vo in steps S030, S060, and S090 are preferably identical to one another.

In periods T1, T3, and T5, the respective lengths of periods T1a, T3a, and T5a during which measuring potential Vm is applied are preferably identical to one another. In periods T1, T3, and T5, the respective lengths of periods T1b, T3b, and T5b during which oxidation potential Vo is applied are preferably identical to one another.

In step S100, the dissolved oxygen concentration in the part of measuring liquid 102 around biological sample 101 is measured based on measured current values I1, I2, and I3.

A method of calculating the dissolved oxygen concentration around biological sample 101 in step S050 based on current value I2 measured in step S050 will be described below.

First, a current change rate I*2 at a certain working electrode 16 among plural working electrodes 16 is determined by dividing the current value I2 measured at the certain working electrode 16 by the current value I1 measured at the certain working electrode 16.

$$\mathrm{I}^*2 = \mathrm{I}2/\mathrm{I}1 \quad (1)$$

By formula (1), the current values at the plural working electrodes 16 are normalized against the current values I1 which possibly be different from each other.

In step S050, oxygen concentration C* consumed by each of the working electrodes is expressed by Formula (2) with dissolved oxygen concentration C in bulk part of measuring liquid 102.

$$\mathrm{C}^* = \mathrm{I}^*2 \times \mathrm{C} = (\mathrm{I}2/\mathrm{I}1) \times \mathrm{C} \quad (2)$$

As described above, in step S050, the dissolved oxygen concentration decreases locally around biological sample 101 due to the oxygen consumed by biological sample 101. The influence of biological sample 101 on the dissolved oxygen in measuring liquid 102 decreases as a distance from biological sample 101 increases.

Accordingly, in step S050, working electrode 16 among plural working electrodes 16 that is closer to biological sample 101 than the other working electrodes 16 exhibits larger current change rate I*2 than those for the other electrodes. In contrast, working electrode 16 among plural working electrodes 16 that is farther from biological sample 101 than the other the working electrodes 16 exhibits smaller current change rate I*2 than the other working electrodes 16.

Current change rate I*2 at working electrode 16 is in inverse proportion to the distance of working electrode 16 from biological sample 101.

Accordingly, oxygen concentration gradient ∆C that appears due to the oxygen consumption by biological sample 101 is expressed by Formula (3) with radius r of biological sample 101 and distance R from the center of biological sample 101 to the center of working electrode 16.

$$\Delta \mathrm{C} = (\mathrm{C} - \mathrm{C}^*) \times \mathrm{R}/\mathrm{r} \quad (3)$$

Distance R may be the distance from the center of biological sample 101 to the center of working electrode 16 when viewed from above. The center of biological sample 101 may be the center of the mounting portion, i.e., region 15.

The respiratory activity of biological sample 101, such as an embryo, having a spherical shape provides an oxygen concentration gradient distributed along a spherical shape about the center of biological sample 101. For this reason, oxygen consumption F which is the total of oxygen fluxes flowing to the surface of spherical biological sample 101 follows the Fick's first law. Consequently, oxygen consumption F is expressed as Formula (4) with diffusion coefficient D of dissolved oxygen in measuring liquid 102.

$$\mathrm{F} = 4 \times \pi \times \mathrm{r} \times \mathrm{D} \times \Delta \mathrm{C} \quad (4)$$

According to the embodiment, since biological sample 101 is placed on region 15 of upper surface 12a of the planar electrode chip, the dissolved oxygen diffuses hemispherically from the center of region 15.

Therefore, according to the embodiment, oxygen consumption F by biological sample 101 is expressed as Formula (5).

$$\mathrm{F} = 2 \times \pi \times \mathrm{r} \times \mathrm{D} \times \Delta \mathrm{C} \quad (5)$$

The dissolved oxygen concentration and oxygen flux in each of steps S020 and S080 is also determined in the above manner.

The surfaces of working electrodes 16 may actually have matters adhered thereto. Such matters induces the decrease of the oxygen-reduction current that flows in each of working electrodes 16 over time.

Even in the blank state, oxygen fluxes may appear in well 14 due to an influence of convection of measuring liquid 102 in well 14.

For this reason, in order to accurately calculate oxygen consumption F by biological sample 101, the oxygen flux determined in step S050 may be corrected based on oxygen fluxes appearing in the blank states in steps S020 and S080.

The current value that flows through working electrode 16 may be measured plural times while biological sample 101 is placed in measuring liquid 102. In this case, the measurement of the current value in the state where biological sample 101 is placed in measuring liquid 102 may be performed plural times in each of steps S050 and S060 alternately and repeatedly between steps S040 and S070.

The electrochemical measuring method described above may be performed in an incubator.

The concentration to be calculated is not limited to the dissolved oxygen concentration. The concentration may be the concentration of another substance that indicates activity of biological sample 101.

In the electrochemical measurement of biological sample 101, a step among steps S010 to S100 in the electrochemical measuring method according to the embodiment may be performed while the other steps may not necessarily performed. For example, current value I3 is not measured in steps S080 to S090 and only current values I1 and I2 are measured in steps S010 to S070. Alternatively, current value I1 is not measured in steps S020 and S030 while only current values I2 and I3 are measured in steps S010 and S040 to S100. Alternatively, current values I1 and I3 are not measured in steps S020, S030, S080, and S090 while only current value I2 is measured in steps S010, S040 to S070, and S100.

As described above, in the performing of the above electrochemical measuring method according to the embodiment, controller 34 of electrochemical measuring apparatus 30 executes: the step of applying measuring potential Vm to working electrode 16 and measuring current value I1 before biological sample 101 is put in; the step of applying oxidation potential Vo to working electrode 16; the step of applying measuring potential Vm to working electrode 16 and measuring current value I2 after biological sample 101 is put in; the step of applying oxidation potential Vo to working electrode 16; and the step of applying measuring potential Vm to working electrode 16 and measuring current value I3 after biological sample 101 is taken out.

Controller 34 does not necessarily execute at least one of the above steps.

In the conventional electrochemical measurement described above, an ambient environment of a biological sample is measured by a method of applying a measuring potential to a working electrode before and after the putting in of the biological sample, or before and after the taking out of the sample. In this method, the measurement performed before and after the putting in of the biological sample, or before and after the taking out of the sample, or the change of the sample over time may cause the measured current which flows through the working electrode upon application of the measuring potential to be unstable, resulting in low reproducibility in the measurement. For this reason, the ambient environment of the biological sample may be hardly measured stably.

As described above, an ambient environment around biological sample 101 is measured by the electrochemical measuring method according to the embodiment.

Modified Example 1 of the Embodiment

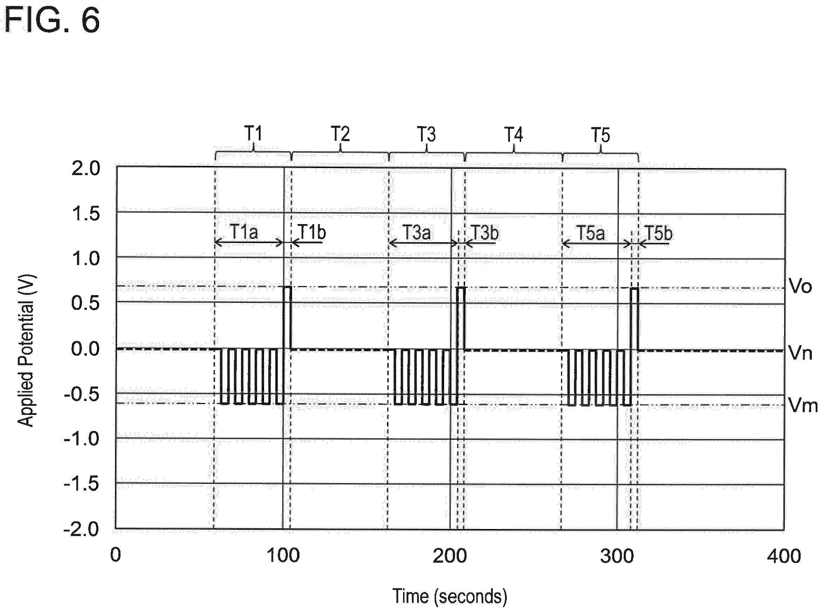

FIG. 6 illustrates another potential application protocol in the electrochemical measurement method shown in FIG. 4 according to the embodiment.

This potential application protocol is different from the potential application protocol shown in FIG. 5 in the way to apply measuring potential Vm. In this potential application protocol, in each of periods T1, T3, and T5 (T1a, T3a, and T5a), measuring potential Vm has a pulse waveform with plural pulses. That is, in each of periods T1a, T3a, and T5a within periods T1, T3, and T5 corresponding to steps S020, S050, and S090, respectively, controller 34 applies measuring potential Vm and non-measuring potential Vn alternately and repeatedly to working electrodes 16. Non-measuring potential Vn is an open-circuit potential of electrochemical measuring apparatus 30. That is, the non-measuring potential is an electric potential of working electrode 16 when working electrode 16 is opened. While non-measuring potential Vn is applied, no current flows through working electrode 16. Non-measuring potential Vn is, e.g. 0.05V in each of the periods, the width of each of the pulses that configure measuring potential Vm, preferably ranges from one (1) second to 10 seconds.

Controller 34 applies, to working electrode 16, measuring potential Vm, which has a pulse waveform configured with measuring potentials Vm and non-measuring potentials Vn in each of periods T1a, T3a, and T5a. Consequently after each of periods T1a, T3a, and T5a, controller 34 applies oxidation potential Vo to working electrode 16 in a corresponding one of periods T1b, T3b, and T5b.

The pulse waveforms of measuring potentials Vm applied during periods T1a, T3a, and T5a are preferably identical to one another. That is, the widths and periods of the plural pulses configuring the pulse waveforms of measuring potentials Vm shown in FIG. 5 are preferably equal to one another.

Measuring potential Vm with the pulse waveform provides current values I1, I2, and I3 of the measured currents to have a pulse waveform. For example, current value I1 obtained by the first pulse of measuring potential Vm in period T1a may allow the other current values I2 and I3 to be normalized. That is, current values I1, I2, and I3 may be replaced with current values I2/I1, and I3/I1, respectively. This configuration provides normalized oxygen-reduction currents.

Measuring potential Vm having the pulsed shape decreases the total time for which measuring potential Vm is applied while biological sample 101 is placed in region 15.

The decreasing of the total time for which measuring potential Vm is applied prevents the reduced substances formed by the oxygen reduction from accumulating on the surface of working electrode 16. This configuration decreases the reduced substances to affect biological sample 101.

In each of periods T1a, T3a, and T5a, controller 34 may apply, to electrode 16, a potential having a pulse waveform that alternately repeats measuring potentials Vm and potentials 0V (zero).

Modified Example 2 of the Embodiment

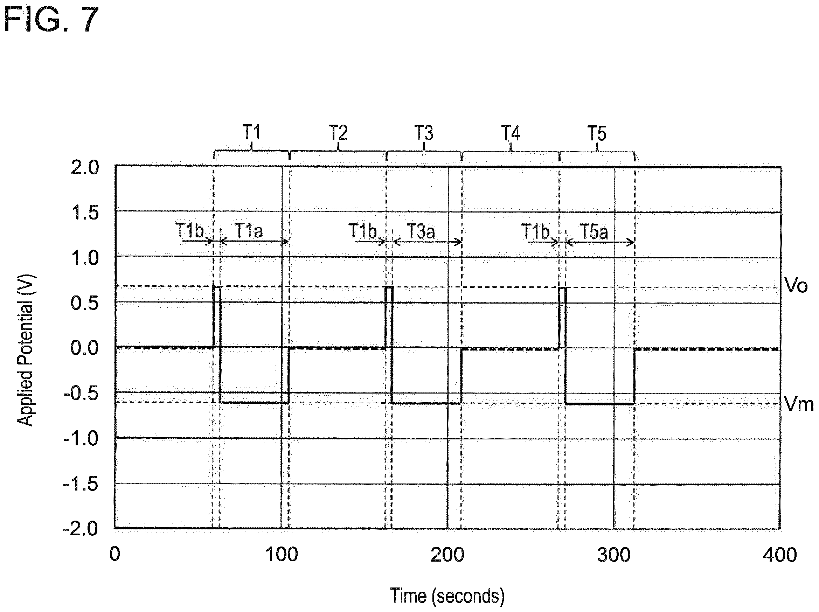

FIG. 7 illustrates still another potential application protocol in the electrochemical measurement method shown in FIG. 4 according to the embodiment.

This potential application protocol is different from the potential application protocol shown in FIG. 5 in the timing of applying oxidation potential Vo in each of periods T1, T3, and T5.

In the potential application protocol shown in FIG. 7, oxidation potential Vo is applied at the beginning of period T1. That is, during period T1, controller 34 applies oxidation potential Vo to working electrodes 16 before applying measuring potential Vm to working electrodes 16. According to the embodiment, oxidation potential Vo is applied to working electrode 16 during period T1b of four (4) seconds lasting from the time point of 60 seconds at which period T1 begins to the time point of 64 seconds. Then, during period T1a subsequent to period T1b, oxidation potential Vo is applied to working electrode 16. Oxidation potential Vo applied to working electrode 16 oxidizes the surface of working electrode 16. This removes substances, such as contaminants, adhered to the surface of working electrode 16 before measuring current value I1. Moreover, this resets the state of the surface of working electrode 16.

In each of periods T3 and T5 as well, oxidation potential Vo and measuring potential Vm are applied in the same manner as in period T1. That is, during period T3, oxidation potential Vo is applied to working electrodes 16 during period T3b starting period T3, and then, oxidation potential Vo is applied to working electrode 16 during period T3a subsequent to period T3b. During period T5, oxidation potential Vo is applied to working electrode 16 during period T5b starting period T5, and then, oxidation potential Vo is applied to working electrode 16 during period T5a subsequent to period T5b.

In cases where no substance, such as contaminants, adheres to the surface of working electrode 16 so that the surface is in a preferable state, oxidation potential Vo is not required to be applied to working electrode 16 in period T1a.

In the potential application protocol shown in FIG. 7, the waveform of measuring potential Vm applied in each of periods T1a, T2a, and T5a may be a pulse waveform configured with plural pulses, in the same manner as in the potential application protocol shown in FIG 6.

Modified Example 3 of the Embodiment

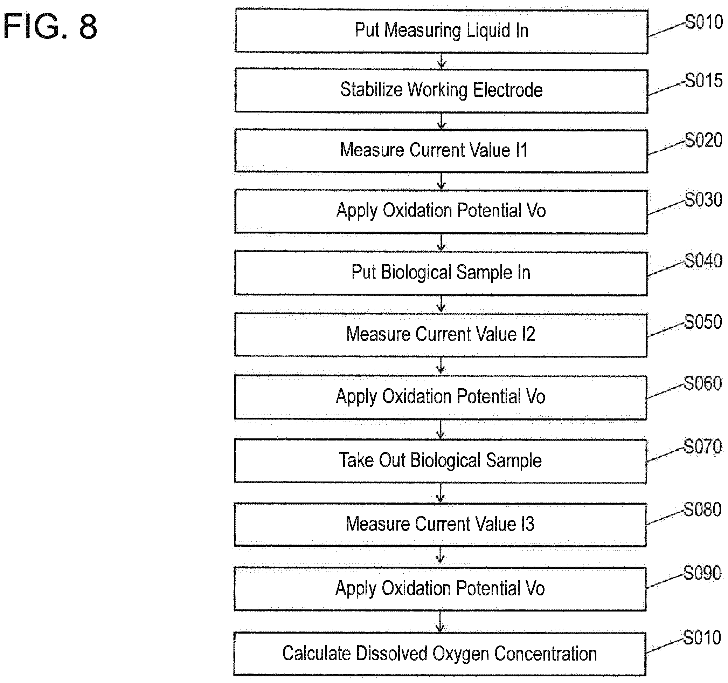

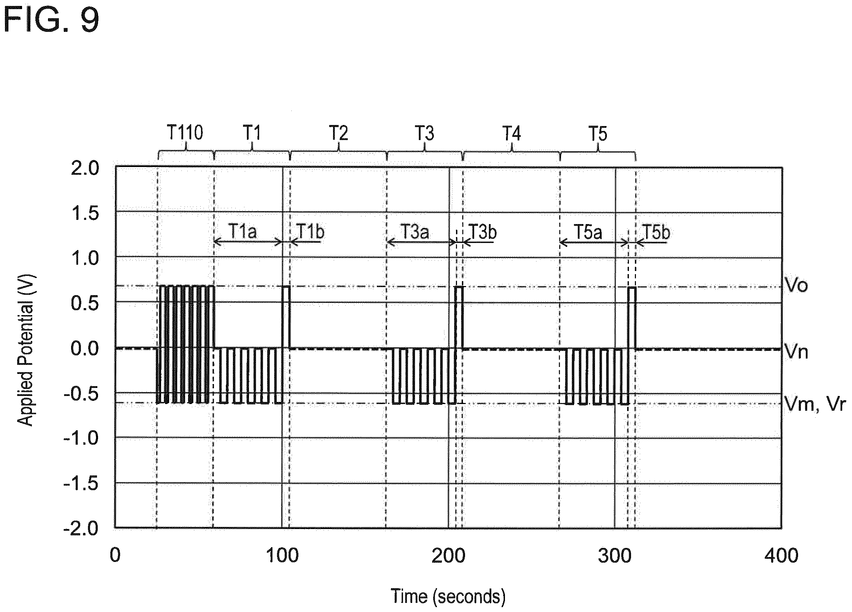

FIG. 8 is a flowchart illustrating another electrochemical measurement method according to the embodiment. FIG. 9 illustrates a potential application protocol in the electrochemical measurement method shown in FIG. 8. In FIGs. 8 and 9, item identical to those of FIGs. 4 and 6 are denoted by the same reference numerals. The electrochemical measuring method shown in FIG. 8 and the potential application protocol shown in FIG. 9 are different from the electrochemical measuring method shown in FIG. 4 and the potential application protocol shown in FIG. 6 in that period T110 is provided further before period T1. During period T110, reduction potential Vr and oxidation potential Vo are applied alternately to each of working electrodes 16.

In the electrochemical measuring method shown in FIG. 8, working electrode 16 is stabilized (step S015) after step 101 of pouring measuring liquid 102 and before step S020 of measuring current value I1. The process in step S015 improves the state of the surface of working electrode 16, resulting in an increase in stability of the current values to be measured.

The process in step S015 is performed as follows. During period T110 preceding period T1, controller 34 applies reduction potential Vr to working electrodes 16. Specifically in step S015, during period T110 preceding period T1, controller 34 applies, to working electrode 16, a pulse waveform configured with plural pulses formed by alternately repeating reduction potential Vr and oxidation potential Vo. Reduction potential Vr is, e.g. -0.6V. Reduction potential Vr and measuring potential Vm may be equal to each other, or different from reach other. Oxidation potential Vo is, e.g. +0.7V.

In the electrochemical measuring method shown in FIG. 8, after the process in step S015 is performed, the steps subsequent to step S020 are performed sequentially in the same manner as in the electrochemical measuring method shown in FIG.4.

Period T110 is required to stabilize working electrodes 16. During period T110, controller 34 may monitor a current value that flows through each of working electrodes 16, thereby determining the time at which the controller applies reduction potential Vr and oxidation potential Vo.

The current value that flows through each of working electrodes 16 is stabilized by applying a potential to working electrode 16 for a while. For this reason, oxidation potential Vo and reduction potential Vr applied to working electrode 16 previously before the measurement of current value I1 decrease variations of current value I1 measured in step S020.

Moreover, the process in step S015 allows abnormality of working electrode 16 and/or electrochemical measuring apparatus 30 to be detected before the measurement in step S020. For example, controller 34 may indicate an error based on the current value measured in step S015.

During period T110 corresponding to step S015, controller 34 may apply, to working electrode 16, a potential that has not necessarily a pulse waveform. For example, during period T110, controller 34 may continuously apply, to working electrode 16, oxidation potential Vo for period T110 that is a predetermined duration.

In the potential application protocol shown in FIG. 9, in each of periods T1a, T2a, and T5a, controller 34 may apply, to working electrode 16, not a pulse waveform but continuous measuring potential Vm.

Modified Example 4 of the Embodiment

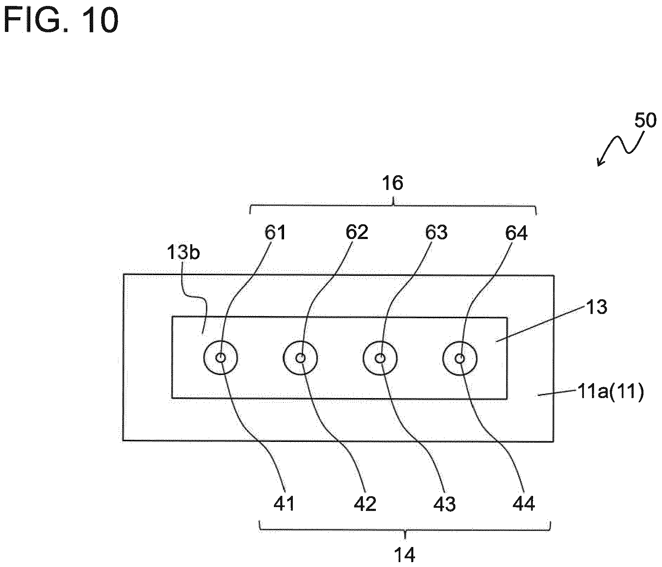

FIG. 10 is a top view of another electrochemical measurement device 50 according to the embodiment. In FIG. 10, components identical to those of electrochemical measuring device 10 shown in FIGs. 1 to 3B are denoted by the same reference numerals.

In electrochemical measurement device 50, plural wells 41, 42, 43, and 44 are provided in bottom surface 13b of reservoir 13. Wells 41, 42, 43, and 44 are collectively called wells 14. The number of wells 14 ranges preferably from two (2) to six (6). Each of wells 14 (41 to 44) has the same structure as well 14 of electrochemical measuring device 10 shown in FIGs. 3A and 3B. Electrode chip 12 is disposed below each of wells 14. Working electrodes 61 are disposed in well 41. Working electrodes 62 are disposed in well 42. Working electrodes 63 are disposed in well 43. Working electrodes 64 are disposed in well 44. Working electrodes 61, 62, 63, and 64 are collectively called working electrodes 16. In electrochemical measurement device 50, plural biological samples 101 each placed in respective one of plural wells 14 can be measured simultaneously in parallel.

Each of biological samples 101 is placed in region 15 in respective one of wells 14.

Electrochemical measurement device 50 operates in the same manner as electrochemical measuring device 10.

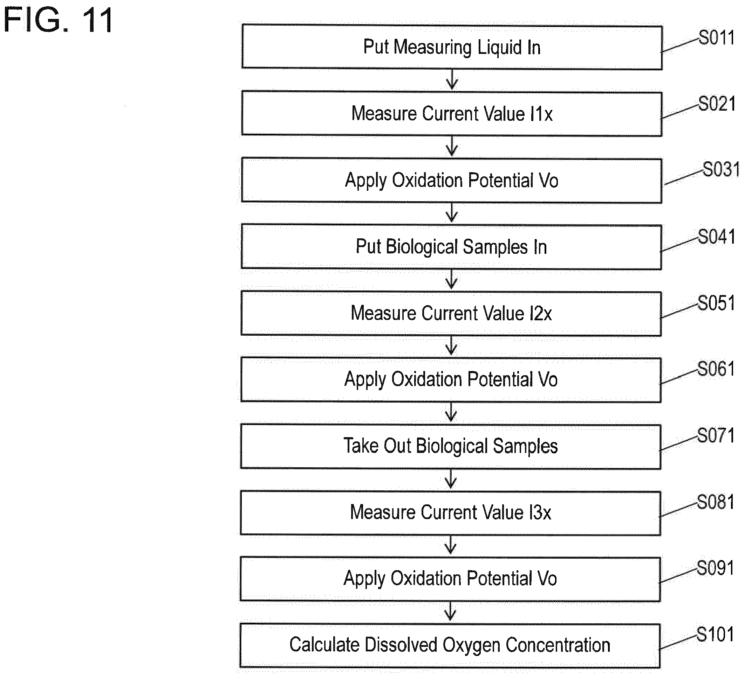

FIG. 11 is a flowchart illustrating an electrochemical measurement method employing electrochemical measurement device 50. A method of measuring the states of activity of plural biological samples 101 according to the modified example will be described below.

In the electrochemical measuring method shown in FIG. 11, measuring liquid 102 is put in container 11 (step S011). Then, in each of wells 14, current value I1x in a blank state in which biological sample 101 is not put in measuring liquid 102 is measured (step S021). Then, oxidation potential Vo is applied to each of working electrodes 16 (step S031). Then, each of plural biological samples 101 is put in respective one of wells 14 (step S041). Then, in each of wells 14, current value I2x is measured while biological sample 101 is put in (step S051). Then, oxidation potential Vo is applied to each of working electrodes 16 (step S061). Then, biological samples 101 are taken out from wells 14 (step S071). Then, in each of wells 14, current value I3x in a blank state after biological samples 101 are taken out is measured (step S081). After that, an amount of a dissolved oxygen concentration in measuring liquid 102 in each of wells 14 based on measured current values I1x, I2x, and I3x (step S101).

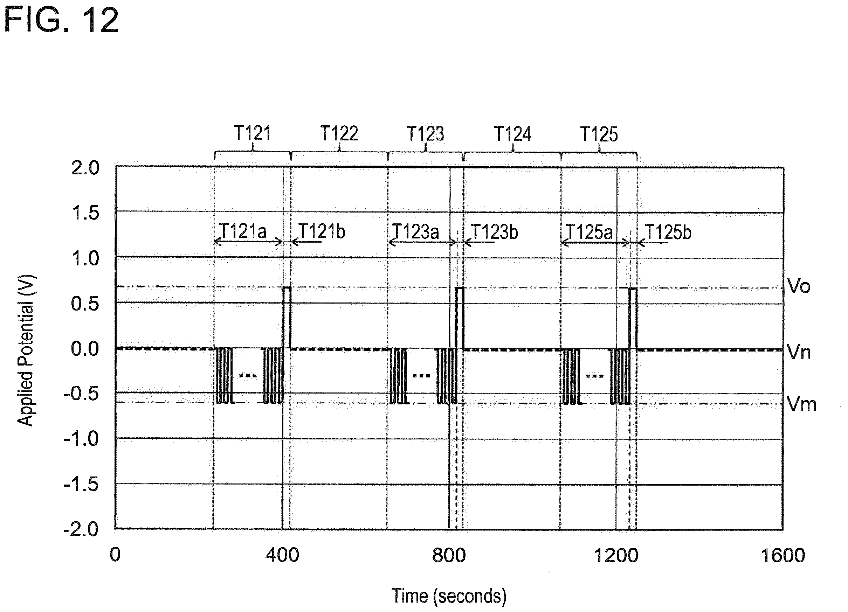

FIG. 12 illustrates a potential application protocol in the electrochemical measurement method shown in FIG. 11. In FIG. 12, the vertical axis represents a potential applied to working electrodes 16, and the horizontal axis represents time.

The electrochemical measuring method shown in FIG. 11 will be detailed below.

In step S011, reservoir 13 of container 11 is charged with measuring liquid 102 to fill a plurality of wells 14 with measuring liquid 102.

In step S021, measuring potential Vm is applied sequentially to each of working electrodes 16 disposed in respective one of wells 14, thereby measuring current value I1x flowing in working electrode 16 in the respective one of respective wells 14 while biological samples 101 are not put in. Current value I1x represents the current flowing through each of wells 14. Measuring potential Vm is applied to working electrodes 16 during period T121.

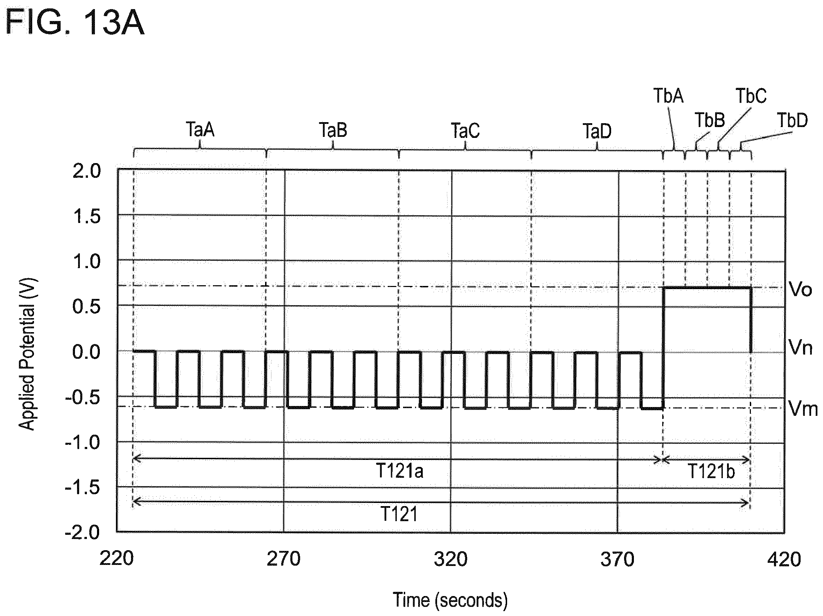

FIG. 13A is an enlarged diagram illustrating period T121 shown in FIG. 12. During period T121 in the potential application protocol shown in FIG. 13A, current value I1x that flows through of working electrodes 16 each of which is located in respective one of wells 14 are measured simultaneously.

Period T121 is configured with period T121a and period T121b subsequent to period T121a. Period T121a is configured with period TaA, period TaB subsequent to period TaA, period TaC subsequent to period TaB, and period TaD subsequent to period TaC. Period T121b is configured with period TbA, period TbB subsequent to period TbA, period TbC subsequent to period TbB, and period TbD subsequent to period TbC.

During period TaA, measuring potential Vm is applied to working electrodes 61 that are disposed in one well 41 among plural wells 14. In accordance with the embodiment, during period TaA, non-measuring potential Vn and measuring potential Vm are applied alternately and repeatedly to working electrode 61. During period TaA, controller 34 may continuously apply measuring potential Vm to working electrode 61 without applying non-measuring potential Vn. This operation allows current value 111 of the current that flows through working electrode 61 in well 41 to be measured. During period TaA, working electrodes 62 to 64 disposed in other wells 42 to 44 among plural wells 14, respectively, electrically are floated from reference electrode 23.

During period TaB, measuring potential Vm is applied to working electrodes 62 disposed in one well 42 among plural wells 14. In accordance with the embodiment, during period TaB, non-measuring potential Vn and measuring potential Vm are applied alternately and repeatedly to working electrode 62. During period TaB, controller 34 may continuously apply measuring potential Vm to working electrode 62 without applying non-measuring potential Vn. This operation allows current value 112 of the current that flows through working electrode 62 in well 42 is measured. During period TaB, working electrodes 61, 63, and 64 respectively disposed in other wells 41, 43, and 44 among plural wells 14 are electrically floated from reference electrode 23.

During period TaC, measuring potential Vm is applied to working electrodes 63 that are disposed in one well 43 among plural wells 14. In accordance with the embodiment, during period TaC, non-measuring potential Vn and measuring potential Vm is alternately and repeatedly applied to working electrode 63. During period TaC, controller 34 may continuously apply measuring potential Vm to working electrode 63 without applying non-measuring potential Vn. This operation allows current value 113 of the current that flows through working electrode 63 in well 43 to be measured. During period TaC, working electrodes 61, 62, and 64 respectively disposed in other wells 41, 42, and 44 among plural wells 14 are electrically floated from reference electrode 23.

During period TaD, measuring potential Vm is applied to working electrodes 64 that are disposed in one well 44 among plural wells 14. In accordance with the embodiment, during period TaD, non-measuring potential Vn and measuring potential Vm are applied alternately and repeatedly to working electrode 64. During period TaD, controller 34 may continuously apply measuring potential Vm to working electrode 64 without applying non-measuring potential Vn. This operation allows current value 114 of the current that flows through working electrode 64 in well 44 to be measured. During period TaD, working electrodes 61 to 63 respectively disposed in other wells 41 to 43 among plural wells 14 are electrically floated from reference electrode 23.

As described above, in each period, measuring potential Vm applied to working electrode 16 has a pulse waveform. In each period, measuring potential Vm applied to working electrode 16 may not necessarily has a pulse waveform but has a constant electric potential.

Working electrode 16 to which measuring potential Vm is applied is thus sequentially switched to another, thereby sequentially measuring current value I1x that flows through corresponding working electrode 16.

In step S031, during period T121b subsequent to period T121a, oxidation potential Vo is applied to working electrodes 16 of each well 14. This operation oxidizes the surfaces of working electrodes 16. Oxidation potential Vo is sequentially applied to working electrodes 16 as follows: After each current value I1x of working electrodes 16 is measured, then the oxidation potential Vo is applied to each of working electrodes 16 in turn.

In more detail, during period TbA subsequent to period TaD that is the last sub-period within period T121a, oxidation potential Vo is applied to working electrodes 61 while the other working electrodes 62 to 64 electrically are floated from reference electrode 23. During period TbB subsequent to period TbA, oxidation potential Vo is applied to working electrodes 62 while the other working electrodes 61, 63, and 64 electrically are floated from reference electrode 23. During period TbC subsequent to period TbB, oxidation potential Vo is applied to working electrodes 63 while the other working electrodes 61, 62, and 64 electrically are floated from reference electrode 23. During period TbD subsequent to period TbC, oxidation potential Vo is applied to working electrodes 64 while the other working electrodes 61 to 63 electrically are floated from reference electrode 23.

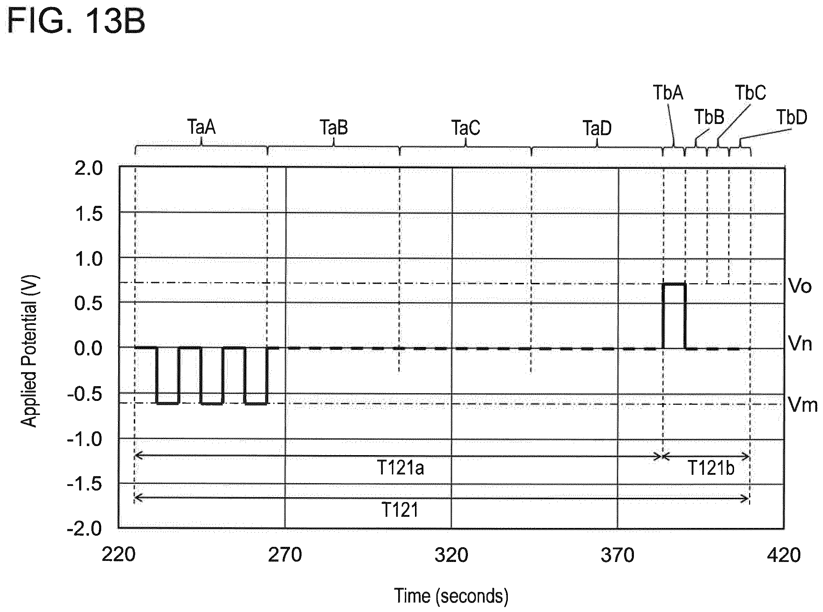

FIG. 13B illustrates the potential applied to each of working electrodes 61 during period T121 in the potential application protocol shown in FIG. 13A. As described above, during period TaA that is the first sub-period within period T121a, controller 34 applies, to working electrode 61, measuring potential Vm and non-measuring potential Vn, alternately and repeatedly, thereby measuring current value 111 of the current that flows through working electrode 61. During periods TaB to TaD subsequent to period TaA, controller 34 causes working electrode 61 to be electrically floated from reference electrode 23. During period TbA that is the first sub-period within period T121b subsequent to period TaD, controller 34 applies oxidation potential Vo to working electrode 61. During periods TbB to TbD subsequent to period TbA, controller 34 causes working electrode 61 to be electrically floated from reference electrode 23. As described above, during period TaA, controller 34 may continuously apply measuring potential Vm to working electrode 61 without applying non-measuring potential Vn.

Controller 34 applies measuring potential Vm, non-measuring potential Vn, and oxidation potential Vo to other working electrodes 62 to 64 as well, at respective different timings that are shifted from the timings for working electrodes 61.

That is, during period TaA, controller 34 causes working electrodes 62 to be electrically floated from reference electrode 23. During period TaB subsequent to period TaA, controller 34 applies, to working electrode 62, measuring potential Vm and non-measuring potential Vn alternately and repeatedly, thereby measuring current value 112 that flows through working electrode 62. During periods TaC and TaD subsequent to period TaB, controller 34 causes working electrode 62 to be electrically floated from reference electrode 23. During period TbA subsequent to period TaD, controller 34 causes working electrode 62 to be electrically floated from reference electrode 23. During period TbB subsequent to period TaA, controller 34 applies oxidation potential Vo to working electrode 62. During periods TbC and TbD subsequent to period TbB, controller 34 causes working electrode 62 to be electrically floated from reference electrode 23. As described earlier, during period TaB, controller 34 may continuously apply measuring potential Vm to working electrode 62 without applying non-measuring potential Vn.

During periods TaA and TaB, controller 34 causes working electrodes 63 to be electrically floated from reference electrode 23. During period TaC subsequent to period TaB, controller 34 applies, to working electrode 62, measuring potential Vm and non-measuring potential Vn alternately and repeatedly, thereby measuring current value I13 that flows through working electrode 63. During period TaD subsequent to period TaC, controller 34 causes working electrode 63 to be electrically floated from reference electrode 23. During periods TbA and TbB subsequent to period TaD, controller 34 causes working electrode 63 to be electrically floated from reference electrode 23. During period TbC subsequent to period TaB, controller 34 applies oxidation potential Vo to working electrode 63. During period TbD subsequent to period TbC, controller 34 causes working electrode 63 to be electrically floated from reference electrode 23. As described above, during period TaC, controller 34 may continuously apply measuring potential Vm to working electrode 63 without applying non-measuring potential Vn.

During periods TaA to TaC, controller 34 causes working electrodes 64 to be electrically floated from reference electrode 23. During period TaD subsequent to period TaC, controller 34 applies, to working electrode 62, measuring potential Vm and non-measuring potential Vn alternately and repeatedly, thereby measuring current value I14 that flows through working electrode 64. During periods TbA to TbC subsequent to period TaD, controller 34 causes working electrode 64 to be electrically floated from reference electrode 23. During period TbD subsequent to period TbC, controller 34 applies oxidation potential Vo to working electrode 64. As described above, during period TaC, controller 34 may continuously apply measuring potential Vm to working electrode 64 without applying non-measuring potential Vn.

In step S041, in period T122, each of plural biological samples 101 is put into respective one of wells 14. In step S041, working electrodes 16 are electrically floated.

In step S051, measuring potential Vm is applied sequentially to working electrodes 16 disposed in respective wells 14, thereby measuring current value I2x with concerned working electrode 16 of a corresponding one of respective wells 14 after biological sample 101 is put in. Here, current value I2x represents the current flowing through each of wells 14. Measuring potential Vm is applied to working electrodes 16 during period T123. In step S051, measuring potential Vm is applied to working electrodes 16 by the same potential applying method as step S021. This allows the measurement of current value I2x.

In step S061, oxidation potential Vo is applied to each working electrode 16 of each well 14. This operation oxidizes the surfaces of working electrodes 16. Oxidation potential Vo is sequentially applied to working electrodes 16 as follows: After current value I2x of each of working electrodes 16 is measured, then the oxidation potential is applied to each of working electrodes 16 in turn. In step S061, oxidation potential Vo is applied to working electrodes 16 by the same potential applying method as step S031. This operation oxidizes the surfaces of working electrodes 16 sequentially.

In step S071, in period T124, biological samples 101 are taken out from wells 14. In step S071, working electrodes 16 are electrically floated.

In step S081, measuring potential Vm is applied sequentially to each of working electrodes 16 that is disposed in respective one of wells 14, thereby measuring current value I3x with concerned working electrode 16 of a corresponding one of wells 14 after biological sample 101 is taken out. Here, current value I3x represents the current flowing through each of wells 14. Measuring potential Vm is applied to working electrodes 16 during period T125. In step S081, measuring potential Vm is applied to working electrodes 16 in the same potential applying method as step S021. This allows the measurement of current value I3x.

During step S091, oxidation potential Vo is applied to working electrodes 16 in wells 14. This operation oxidizes the surfaces of working electrodes 16. Oxidation potential Vo is sequentially applied to working electrodes 16 as follows: After current value I2x of each of working electrodes 16 is measured, then the oxidation potential is applied to each of working electrodes 16 in turn. In step S061, oxidation potential Vo is applied to working electrodes 16 in the same potential applying method as step S031. This operation oxidizes the surfaces of working electrodes 16 sequentially.

After step S081, in cases where the measurement of a current value by applying an electric potential to working electrodes 16 is not performed, step S091 is not required to be performed because of no need for holding the condition of surfaces of working electrodes 16.

In step S101, based on measured current values I1x, I2x, and I3x, amount of a dissolved oxygen concentration in measuring liquid 102 in each of wells 14 is calculated.

In steps S031, S061, and S091, before or after the current value is measured for each well, oxidation potential Vo may be applied to plural wells 14 at once.

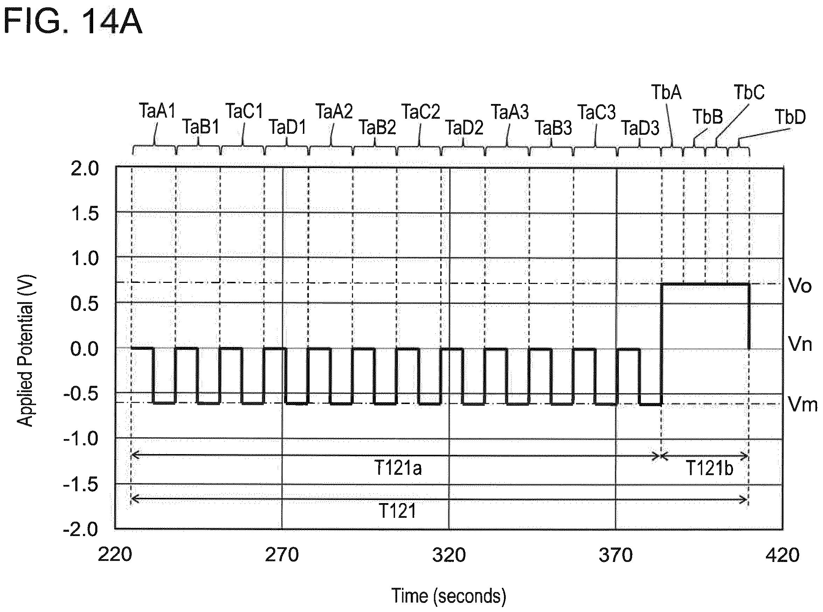

FIG. 14A is an enlarged diagram illustrating period T121 of another potential application protocol in the electrochemical measurement method shown in FIGs. 11 and 12. In FIG. 14A, the vertical axis represents a potential applied to working electrodes 16, and the horizontal axis represents time. In the potential application protocol shown in FIG. 14A, period T121a is configured with periods TaA1, TaB1, TaC1, TaD1, TaA2, TaB2, TaC2, TaD2, TaA3, TaB3, TaC3, and TaD3 which succeed in this order.

In accordance with the potential application protocol shown in FIG. 14A, in steps S021, S051, and S081 shown in FIG. 11, the operation of applying measuring potential Vm sequentially to working electrodes 16 disposed in the plurality of wells 14, is repeated several times. The configuration allows the measurements of current values I1x, I2x, and I3x. That is, for measuring the current values in these steps, periods TaA to TaD are repeated several times. The operation will be detailed below.

During periods TaA1, TaA2, and TaA3, measuring potential Vm is applied to working electrodes 61 that are disposed in one well 41 among plural wells 14. In accordance with the embodiment, during periods TaA1, TaA2, and TaA3, measuring potential Vm is applied after non-measuring potential Vn is applied. In periods TaA1, TaA2, and TaA3, controller 34 may continuously apply measuring potential Vm to working electrodes 61 without applying non-measuring potential Vn. The configuration allows the measurement of current value I11 that flows through each of working electrodes 61 in corresponding well 41. During periods TaA1, TaA2, and TaA3, working electrodes 62 to 64 disposed in other wells 42 to 44 among the plural wells 14 are electrically floated from reference electrode 23.

During periods TaB1, TaB2, and TaB3 respectively subsequent to periods TaA1, TaA2, and TaA3, measuring potential Vm is applied to working electrodes 62 disposed in one well 42 among the plural wells 14. In accordance with the embodiment, during periods TaB1, TaB2, and TaB3, measuring potential Vm is applied after non-measuring potential Vn is applied. During periods TaB1, TaB2, and TaB3, controller 34 may continuously apply measuring potential Vm to working electrodes 62 without applying non-measuring potential Vn. The configuration allows the measurement of current value I12 that flows through each of working electrodes 62 in corresponding well 42. During periods TaB1, TaB2, and TaB3, working electrodes 61, 63, and 64 respectively disposed in other wells 41, 43, and 44 among the plural wells 14 are electrically floated from reference electrode 23.

During periods TaC1, TaC2, and TaC3 respectively subsequent to periods TaB1, TaB2, and TaB3, measuring potential Vm is applied to working electrodes 63 disposed in one well 43 among the plural wells 14. In accordance with the embodiment, during periods TaC1, TaC2, and TaC3, measuring potential Vm is applied after non-measuring potential Vn is applied. During periods TaC1, TaC2, and TaC3, controller 34 may continuously apply measuring potential Vm to working electrodes 63 without applying non-measuring potential Vn. The configuration allows the measurement of current value I13 that flows through each of working electrodes 63 in corresponding well 43. During periods TaC1, TaC2, and TaC3, working electrodes 61, 62, and 64 respectively disposed in other wells 41, 42, and 44 among the plural wells 14 are electrically floated from reference electrode 23.

During periods TaD1, TaD2, and TaD3 respectively subsequent to periods TaC1, TaC2, and TaC3, measuring potential Vm is applied to working electrodes 64 disposed in one well 44 among the plural wells 14. In accordance with the embodiment, during periods TaD1, TaD2, and TaD3, measuring potential Vm is applied after non-measuring potential Vn is applied. During periods TaD1, TaD2, and TaD3, controller 34 may continuously apply measuring potential Vm to working electrodes 64 without applying non-measuring potential Vn. The configuration allows the measurement of current value I14 that flows through each of working electrodes 64 in corresponding well 44. During periods TaD1, TaD2, and TaD3, working electrodes 61 to 63 respectively disposed in other wells 41 to 43 among the plural wells 14 are electrically floated from reference electrode 23.

In the potential application protocol shown in FIG. 14A, during period T121b, controller 34 applies oxidation potential Vo to working electrodes 26 (61 to 64) in the same manner as the potential application protocol shown in FIGs. 13A and 13B.

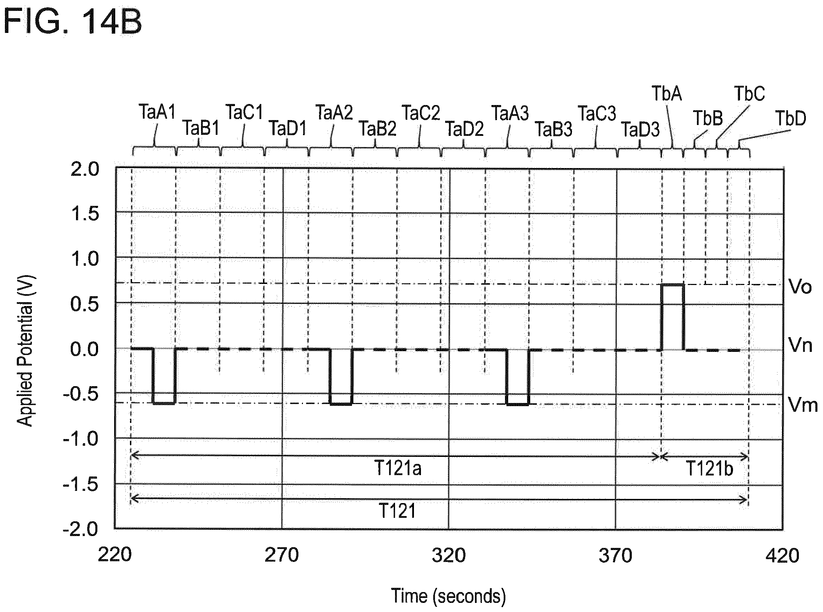

FIG. 14B illustrates the electric potential applied to working electrode 61 in period T121 in the potential application protocol shown in FIG. 14A. As described above, during the first periods TaA1, TaA2, and TaA3 within period T121a, controller 34 applies, to each working electrode 61, measuring potential Vm and non-measuring potential Vn alternately and repeatedly, thereby measuring current value I11 that flows through working electrode 61. During periods TaB1 to TaD1 respectively subsequent to periods TaA1, TaA2, and TaA3, controller 34 causes working electrode 61 to be electrically floated from reference electrode 23. During period TbA that is the first sub-period in period T121b subsequent to period TaD3, controller 34 applies oxidation potential Vo to working electrode 61. During periods TbB to TbD subsequent to period TbA, controller 34 causes working electrode 61 to be electrically floated from reference electrode 23. As described earlier, during periods TaA1, TaA2, and TaA3, controller 34 may continuously apply measuring potential Vm to working electrode 61 without applying non-measuring potential Vn.

Controller 34 applies measuring potential Vm, non-measuring potential Vn, and oxidation potential Vo to other working electrodes 62 to 64 as well, at respective different timings shifted from the timings for working electrode 61.

That is, during periods TaA1, TaA2, and TaA3, controller 34 causes working electrodes 62 to be electrically floated from reference electrode 23. During periods TaB1, TaB2, and TaB3 respectively subsequent to periods TaA1, TaA2, and TaA3, controller 34 alternately applies measuring potential Vm and non-measuring potential Vn to working electrode 62, thereby measuring current value I12 that flows through working electrode 62. During periods TaC1, TaD1, TaC2, TaD2, TaC3, and TaD3 respectively subsequent to periods TaB1, TaB2, and TaB3, controller 34 causes working electrode 62 to be electrically floated from reference electrode 23. During period TbA subsequent to period TaD3, controller 34 causes working electrode 62 to be electrically floated from reference electrode 23. During period TbB subsequent to period TaA, controller 34 applies oxidation potential Vo to working electrode 62. During periods TbC and TbD subsequent to period TbB, controller 34 causes working electrode 62 to be electrically floated from reference electrode 23. As described earlier, during period TaB, controller 34 may continuously apply measuring potential Vm to working electrode 62 without applying non-measuring potential Vn.

During periods TaA1, TaA2, TaA3, TaB1, TaB2, and TaB3, controller 34 causes working electrodes 63 to be floated from reference electrode 23. During periods TaC1, TaC2, and TaC3 respectively subsequent to periods TaB1, TaB2, and TaB3, controller 34 applies measuring potential Vm and non-measuring potential Vn to working electrode 62 alternately and repeatedly, thereby measuring current value 113 that flows through working electrode 63. During periods TaD1, TaD2, and TaD3 respectively subsequent to periods TaC1, TaC2, and TaC3, controller 34 causes working electrode 63 to be electrically floated from reference electrode 23. In periods TbA and TbB subsequent to period TaD3, controller 34 causes working electrode 63 to be electrically floated from reference electrode 23. During period TbC subsequent to period TaB, controller 34 applies oxidation potential Vo to working electrode 63. During period TbD subsequent to period TbC, controller 34 causes working electrode 63 to be electrically floated from reference electrode 23. As described above, during periods TaC1, TaC2, and TaC3, controller 34 may continuously apply measuring potential Vm to working electrode 63 without applying non-measuring potential Vn.

During periods TaA1, TaA2, TaA3, TaB1, TaB2, TaB3, TaC1, TaC2, and TaC3, controller 34 causes working electrodes 64 to be electrically floated from reference electrode 23. During periods TaD1, TaD2, and TaD3 respectively subsequent to periods TaC1, TaC2, and TaC3, controller 34 applies measuring potential Vm and non-measuring potential Vn to working electrode 62 alternately and repeatedly, thereby measuring current value 114 that flows through working electrode 64. During periods TbA to TbC subsequent to period TaD3, controller 34 causes working electrode 64 to be electrically floated from reference electrode 23. During period TbD subsequent to period TaC, controller 34 applies oxidation potential Vo to working electrode 64. As described above, during periods TaC1, TaC2, and TaC3, controller 34 may continuously apply measuring potential Vm to working electrode 64 without applying non-measuring potential Vn.

Electrochemical measuring apparatus 30 applies measuring potential Vm to working electrodes 16 of each well 14 such that the working electrode to which the measuring potential is applied is switched to another every one pulse of the measuring potential. Switching of well 14 to another every one pulse in this way, allows time intervals between the measurements for wells 14 to be approximately equal to one another; such measurements include: the blank measurements in steps S021 and S081, and the activity measurements of biological samples in step S051. During each of the sub-periods in one operation, the number of pulses of measuring potential Vm applied to working electrode 16 is not limited to one (1), but may be two (2) or more.

The sum of the numbers of pulses of measuring potential Vm is equal to an integral multiple of the number of biological samples 101 to be measured.

The number and widths of pulses of measuring potential Vm applied to one working electrode 61 are preferably equal to the numbers and widths of pulses of measuring potentials Vm applied to other working electrodes 62, 63, and 64.

In the embodiment described above, in measuring a dissolved oxygen concentration, measuring potential Vm is equal to reduction potential Vr. However, in other cases of electrochemical measurements, measuring potential Vm may be set equal to oxidation potential Vo. In this case, reduction potential Vr may be applied to working electrodes 16 in place of oxidation potential Vo. That is, in the potential application protocol described above, measuring potential Vm and oxidation potential Vo are replaced with each other, thereby allowing the surfaces of working electrodes 16 to be held in a constant state.

Although the electrochemical measuring method has been described according to one or more aspects based on the aforementioned exemplary embodiments, the present disclosure is obviously not limited to such exemplary embodiments. Other forms in which various modifications apparent to those skilled in the art are applied to any of the aforementioned exemplary embodiments, or forms structured by combining structural elements of different aspects of the embodiments may be included within the scope of the one or more aspects, unless such changes and modifications depart from the scope of the present disclosure.

In the embodiments described above, terms, such as "upper surface," lower surface," "upper container," and "lower container," indicating directions merely indicate relative directions depending only on the relative positional relationship among the constituent components, such as the electrode chips and the containers, of the electrochemical measuring device, and do not indicate absolute directions, such as a vertical direction.

REFERENCE MARKS IN THE DRAWINGS

| 10, 50 | electrochemical measuring device |
| 11 | container |
| 12 | electrode chip |
| 13 | reservoir |
| 14, 41-44 | well |
| 15 | region |
| 16, | 61-64 working electrode |
| 17 | connection terminal |
| 18 | sealing member |
| 101 | biological sample |
| 102 | measuring liquid |
| 23 | reference electrode |
| 24 | counter electrode |
| 30 | electrochemical measuring apparatus |
| 33 | terminal |
| 34 | controller |
| 35 | cover |
| 36 | measurement unit |
| 37 | calculating unit |

**Claims**

1. A method for electrochemically measuring a state of a biological sample, comprising:

   preparing an electrochemical measuring device including a working electrode, the electrochemical measuring device being filled with a measuring liquid contacting the working electrode;
   putting the biological sample into the measuring liquid;
   measuring a current value flowing through the working electrode by applying a measuring potential to the working electrode; and
   oxidizing a surface of the working electrode by applying an oxidation potential to the working electrode.

2. The method of claim 1,

   wherein said measuring of the current value flowing through the working electrode comprises:

   measuring a first current value flowing through the working electrode by applying the measuring potential to the working electrode before said putting of the biological sample into the measuring liquid; and
   measuring a second current value flowing through the working electrode by applying the measuring potential to the working electrode while the biological sample is in the measuring liquid, and

   wherein said oxidizing of the surface of the working electrode comprises oxidizing the surface of the working electrode by applying the oxidation potential to the working electrode after said measuring of the first current value.

3. The method of claim 2, further comprising

   taking out the biological sample from the measuring liquid,
   wherein said oxidizing of the surface of the working electrode further comprises oxidizing the surface of the working electrode by applying the oxidation potential to the working electrode after said measuring of the second current value, and
   wherein said measuring of the current value of the electric current flowing through the working electrode further comprises measuring a third current value flowing through the working electrode by applying the measuring potential to the working electrode after said taking out of the biological sample from the measuring liquid.

4. The method of claim 3, wherein said oxidizing of the surface of the working electrode further comprises oxidizing the surface of the working electrode by applying the oxidation potential to the working electrode after said measuring of the third current value.

5. The method of claim 2, further comprising oxidizing the surface of the working electrode by applying the oxidation potential to the working electrode before said measuring of the first current value.

6. The method of claim 5, wherein said oxidizing of the surface of the working electrode by applying the oxidation potential to the working electrode before said measuring of the first current value comprises alternately applying, to the working electrode, the oxidation potential and a reduction potential reducing the working electrode before said measuring of the first current value.

7. The method of claim 1, wherein the measuring potential has a pulse waveform.

8. The method of claim 1, wherein the measuring potential is a negative potential, and the oxidation potential is a positive potential.

9. The method of claim 8,

   wherein the electrochemical measuring device further includes a reference electrode,

wherein said preparing of the electrochemical measuring device comprises preparing the electrochemical measuring device filled with the measuring liquid contacting both the working electrode and the reference electrode,

wherein the measuring potential is a negative potential with respect to the reference electrode, and

wherein the oxidation potential is a positive potential with respect to the reference electrode.

10. A method for electrochemically measuring a state of a biological sample, the method comprising:

preparing an electrochemical measuring device including a working electrode, the electrochemical measuring device being filled with a measuring liquid contacting the working electrode;

putting the biological sample into the measuring liquid;

measuring a current value flowing through the working electrode by applying a measuring potential to the working electrode; and

reducing a surface of the working electrode by applying a reduction potential to the working electrode.

11. An electrochemical measuring apparatus comprising:

an electrochemical measuring device which includes a well and a working electrode disposed in the well;

a terminal electrically connected to the working electrode of the electrochemical measuring device; and

a controller configured to

measure a current value flowing through the working electrode by applying a measuring potential to the working electrode for measuring the current value flowing through the working electrode, and

oxidize a surface of the working electrode by applying an oxidation potential to the working electrode for oxidizing the surface of the working electrode.

12. The electrochemical measuring apparatus of claim 11, further comprising a cover that covers the electrochemical measuring device.

FIG. 1

EP 3 553 511 A1

FIG. 2

FIG. 3A

# FIG. 3B

EP 3 553 511 A1

# FIG. 4

| | |
|---|---|
| Put Measuring Liquid In | S010 |
| ↓ | |
| Measure Current Value I1 | S020 |
| ↓ | |
| Apply Oxidation Potential Vo | S030 |
| ↓ | |
| Put Biological Sample In | S040 |
| ↓ | |
| Measure Current Value I2 | S050 |
| ↓ | |
| Apply Oxidation Potential Vo | S060 |
| ↓ | |
| Take Out Biological Sample | S070 |
| ↓ | |
| Measure Current Value I3 | S080 |
| ↓ | |
| Apply Oxidation Potential Vo | S090 |
| ↓ | |
| Calculate Dissolved Oxygen Concentration | S100 |

FIG. 5

EP 3 553 511 A1

FIG. 6

# FIG. 7

EP 3 553 511 A1

# FIG. 8

| | |
|---|---|
| Put Measuring Liquid In | S010 |
| Stabilize Working Electrode | S015 |
| Measure Current Value I1 | S020 |
| Apply Oxidation Potential Vo | S030 |
| Put Biological Sample In | S040 |
| Measure Current Value I2 | S050 |
| Apply Oxidation Potential Vo | S060 |
| Take Out Biological Sample | S070 |
| Measure Current Value I3 | S080 |
| Apply Oxidation Potential Vo | S090 |
| Calculate Dissolved Oxygen Concentration | S010 |

EP 3 553 511 A1

FIG. 9

# FIG. 10

# FIG. 11

| | |
|---|---|
| Put Measuring Liquid In | S011 |
| Measure Current Value I1x | S021 |
| Apply Oxidation Potential Vo | S031 |
| Put Biological Samples In | S041 |
| Measure Current Value I2x | S051 |
| Apply Oxidation Potential Vo | S061 |
| Take Out Biological Samples | S071 |
| Measure Current Value I3x | S081 |
| Apply Oxidation Potential Vo | S091 |
| Calculate Dissolved Oxygen Concentration | S101 |

FIG. 12

FIG. 13A

# FIG. 13B

# FIG. 14A

EP 3 553 511 A1

# FIG. 14B

EP 3 553 511 A1

## INTERNATIONAL SEARCH REPORT

International application No.

PCT/JP2017/042716

### A. CLASSIFICATION OF SUBJECT MATTER

Int.Cl. G01N27/416(2006.01)i, C12M1/34(2006.01)i, C12Q1/02(2006.01)i, G01N27/28(2006.01)i

According to International Patent Classification (IPC) or to both national classification and IPC

### B. FIELDS SEARCHED

Minimum documentation searched (classification system followed by classification symbols)
Int.Cl. G01N27/416, C12M1/34, C12Q1/02, G01N27/28

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| X<br>Y | WO 2016/047114 A1 (PANASONIC INTELLECTUAL PROPERTY MANAGEMENT CO., LTD.) 31 March 2016, claims, paragraphs [0001]–[0003], [0015]–[0075], fig. 1–6<br>& US 2017/0218423 A1, paragraphs [0001]–[0003], [0028]–[0087], fig. 1–6 & EP 3199637 A1 | 1–5, 7–9, 11–12<br>6, 10 |
| Y | JP 2016–501070 A (MEDTRONIC MINIMED, INC.) 18 January 2016, paragraphs [0011], [0107], fig. 10<br>& US 2014/0135605 A1, paragraphs [0010], [0125], fig. 10 & WO 2014/078409 A1 & CN 104853674 A | 6 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 January 2018 | 06 February 2018 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.

PCT/JP2017/042716

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2000-171437 A (SAWAMOTO, Isao) 23 June 2000, paragraphs [0010], [0011] (Family: none) | 10 |
| A | WO 2010/055942 A1 (CLINO CORPORATION) 20 May 2010, entire text, all drawings & JP 2010-121948 A | 1-12 |
| A | JP 2009-533690 A (DIONEX CORPORATION) 17 September 2009, entire text, all drawings & US 2007/0240998 A1 & WO 2007/121267 A2 | 1-12 |
| A | JP 2008-64661 A (ALOKA CO., LTD.) 21 March 2008, entire text, all drawings (Family: none) | 1-12 |
| A | SHIKU, Hitoshi et al., Respiration activity of single bovine embryos entrapped in a cone-shaped microwell monitored by scan, Analytica Chimica Acta, 2004, vol. 522, pp. 51—58 | 1-12 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010055942 A